# EUROPEAN PATENT SPECIFICATION

(11) **EP 1 147 743 B1**
(45) Date of publication and mention of the grant of the patent: **07.09.2005**
(21) Application number: 00850069.6
(22) Date of filing: 19.04.2000
(51) Int. Cl.: A61B 17/00

(54) **Intra-arterial occluder**
Intraarterielle Okklusionsvorrichtung
Dispositif d'occlusion artérielle

(43) Date of publication of application: 24.10.2001
(73) Proprietor: RADI MEDICAL SYSTEMS AB, 754 50 Uppsala (SE)
(72) Inventor: Akerfeldt, Dan, 755 92 Uppsala (SE); Preinitz, Frederik, 753 50 Uppsala (SE); Egnelov, Per, SE-754 40 Uppsala (SE)
(74) Representative: Lindgren, Anders

(56) References cited:
- EP-A- 0 894 475
- US-A- 4 744 364
- US-A- 5 620 461
- US-A- 5 725 577

## Description

### Technical Field of the Invention

The present invention relates to a plug for sealing a percutaneous puncture in a vessel, at the inner surface of the vessel.

### Description of the Prior Art

During certain types of medical surgery or treatment an introducer is used to access the vascular system of a patient. The introducer is inserted through the wall of a blood vessel in order to obtain access to the vascular system and may thereafter be used for guiding medical instruments such as catheters, guide wires and the like.

After the completion of the medical procedure there will be an incision or a wound in the wall of the blood vessel corresponding to the size of the introducer. The bleeding from the wound, which is the result of such a surgical operation, may be stopped by applying direct pressure on the wound. However, applying direct pressure on the wound will require assistance of medical personnel and may also restrict the blood flow through the vessel.

EP-766 947 A2 describes a hemostatic puncture device for sealing a percutaneous puncture. The main parts of this device are an anchoring means, a collagen foam acting as a sealing means, a filament means and carrier means. The device uses an introducer or the like in order to guide the different parts to the puncture. The anchoring means, which is a narrow, rigid beam member, is introduced through the puncture to be inserted into the vessel. During the introduction, the anchoring means is in a longitudinal position, in order to fit into the introducer. In order to function as an anchor the anchoring means is manipulated in such a way that its end portions grip the inner edges of the puncture. The anchoring means is connected to the sealing means by the filament means in a pulley-like configuration. Thus, after the anchoring means has been put in place and the introducer is withdrawn the pulley-like configuration will pull the sealing means towards the puncture and then eventually seal the puncture on the outside wall of the vessel. Thus, the collagen foam performs all the sealing, i.e. the puncture is only sealed on the outside wall of the vessel. The collagen foam is effective in stopping the flow of blood, but the closure device according to EP-766 947 has disadvantages. One such disadvantage is the risk that the local tension applied to the edges of the puncture by the anchoring means, which contacts the puncture edge at two sites only, will rupture the edges of the puncture. In addition, the use of a sealing that seals on the outside of the vessel requires higher sealing force than a corresponding inner sealing.

In EP 0894475 a temporary intravascular arteriotomy seal for insertion into and retrieval from a blood vessel through an opening in the wall of said vessel is described. It is adapted to be folded in the width direction upon exertion of a force on the gripping element directed in the length direction, and upon contacting of the sheet material by the sides of the opening in the vessel wall.

Through US 5,350,399, which is assessed to be the closest prior art, it is known to seal a puncture through a vessel with an intra-arterial occluder and an extra-arterial occluder, respectively. The occluders are made of resilient biocompatible and/or bioabsorbable material and are held together by a saw-toothed guide extending from the intra-arterial occluder. A similar sealing is also disclosed in US 5,342,393 wherein inner and outer rivet members are joined by a stem extending from the inner rivet to seal a puncture.

In addition, an intra-arterial occluder is described in US patent no. 4,852,568.

However, when using an intra-arterial occluder there is still a problem in that the edge of the puncture might rupture when a retracting force is applied to the stem of the intra-arterial occluder in order to urge it against the vessel wall. In addition, the occluder according to US 5,342,393 includes small hook-like means to clamp the edge of the puncture in order to ensure the sealing function of the occluders. These hook-like means are also a possible source for damage to the edge of the puncture.

Thus there is a need for an improved intra-arterial occluder that provides a safe sealing of a percutaneous puncture, and at the same time reduces the risk of a rupture in the vessel wall when applying a retracting force to the intra-arterial occluder in order to urge the occluder against the vessel wall.

### Summary of the Invention

According to the present invention, this object is achieved with an intra-arterial occluder according to claim 1 of the appended claims.

The intra-arterial occluder according to the invention exhibits a central elongated portion, attached to e retracting means, said central elongated portion having a higher stiffness than a surrounding rim portion, in order to transfer the retracting force to those areas surrounding the puncture that have the highest strength against rupture. In a preferred embodiment, the higher stiffness is achieved by forming the central portion with a considerably higher thickness than the surrounding rim portion.

In use, the occluder is passed through the puncture, and is oriented such that the axis of the central portion is generally coaxial with the vessel. When retracted, due to its stiffness the central portion will rest against the vessel wall at two diametrically opposed areas around the puncture, and along the length of the vessel. These areas represent the strongest areas around the puncture, and will therefore be able to safely carry the retracting force. At the same time, the less stiff circumferential rim portion, which is easily foldable to fit into an insertion tool, adapts to the vessel wall and acts as an efficient sealing.

Further scope of applicability of the present invention will become apparent from the detailed description given hereinafter. However, it should be understood that the detailed description and specific examples, while indicating preferred embodiments of the invention are given by way of illustration only. Various changes and modifications within the scope of the invention will become apparent to those skilled in the art from this detailed description. Reference is made to the accompanying drawings, which are given by way of illustration only and thus are not limiting the present invention, wherein
- Fig. 1: is a front view of an embodiment of an intra-arterial occluder according to the present invention.
- Fig. 2: is a cross sectional view taken along line I-I of Fig. 1.
- Fig. 3: is a side view of the occluder of Fig. 1.
- Fig. 4: is a schematic illustration of a puncture in a vessel.
- Fig. 5: is a cross sectional view, perpendicular to the vessel, of an intra-arterial occluder according to the present invention sealing a puncture.
- Fig. 6: is a cross sectional view, along the length of the vessel, of an intra-arterial occluder according to the present invention sealing a puncture.
- Fig. 7: shows another embodiment of an occluder according to the invention.
- Fig. 8: is a cross section of an introducer and an occluder disposed therein.
- Fig. 9: is a front view of a second embodiment of an intra-arterial occluder according to the present invention.
- Fig. 10: is a cross sectional view taken along line II-II of Fig. 9.

### Detailed Description of Preferred Embodiments

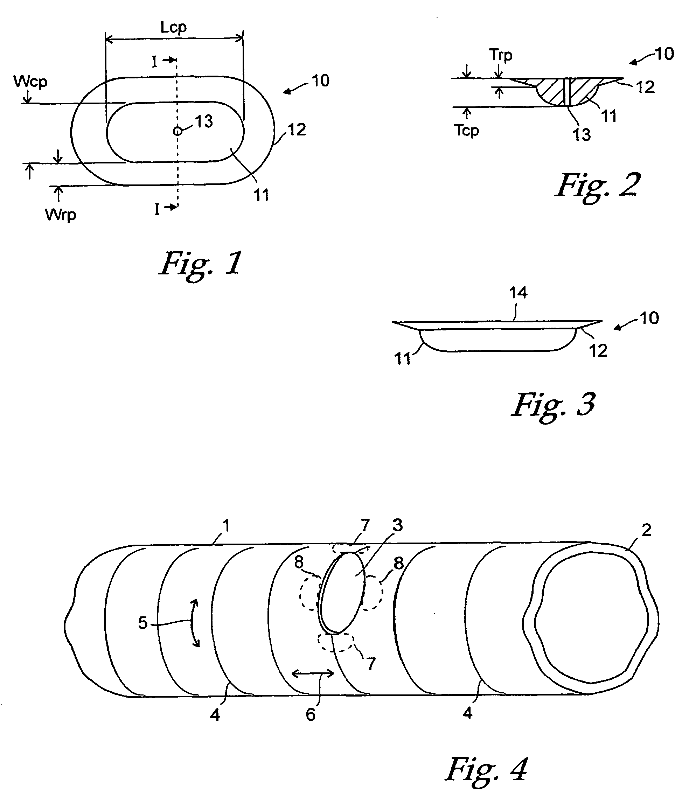

In Fig. 4 is shown a portion of a vessel 1 in a living body, such as the femoral artery. A puncture 3 has been made through the vessel wall 2, thereby creating an opening which has to be occluded after the treatment which made the puncture necessary. Indicated in Fig. 4 is also the fibrous structure 4 of the vessel. This fibrous structure has the effect that the vessel is relatively strong in its circumferential direction 5. At the same time, the strength of the vessel fluctuates and is comparatively weak in its longitudinal direction 6.

Therefore, when applying a retracting force (i.e. a force acting on the edge of the puncture and being directed outward from the vessel) those areas 8 near the edge of the rupture that are situated along the axis of the vessel can withstand a higher tension than those areas 7 that are situated transversally thereto.

According to the invention, this circumstance can be utilized to form an improved intra-arterial occluder, an embodiment of such an occluder 10 being shown in Figs. 1, 2 and 3, wherein Fig. 1 is a front view, Fig. 2 is a cross sectional view and Fig. 3 is a side view.

In general, the occluder 10 according to the invention comprises an elongated comparatively stiff central portion 11, surrounded by a rim portion 12 on at least the longitudinal sides of the central portion, said rim portion having a reduced stiffness with respect to the central portion. In addition, the occluder is provided with a generally flat surface 14, extending over the central portion and the rim portion, for contact with the inner wall of the vessel around the puncture, although this surface also could be provided with a certain degree of texture.

The dimensions of the occluder should be selected to suit the puncture to be sealed. As a guidance for practicing the present invention, some general recommendation on occluder dimensions shall now be given.

The central portion 11 has a longitudinal length Lcp that exceeds the diameter of the puncture in the vessel direction, taking the dimensions of the occluder introducing tool (as will be described below) into account since it is probable that the occluder inserting tool will widen the puncture to some extent. Preferably, the length Lcp of the central portion is about 50 % longer than the diameter of the puncture in the vessel direction. A typical value of Lcp is about 6 mm.

At the same time, the width Wcp of the central portion should be less than the puncture diameter in the direction transverse to the vessel direction, in order to be safely inserted through the puncture in a folded state and to reduce the width of the abutment areas of the central portion against the vessel wall.

The rim portion 12 of lower stiffness could be formed as separate portions extending along each side of the central portion, although they are preferably formed as a surrounding continuous rim around the central portion (as shown in the figures).

In a preferred embodiment, as will be described below, the different stiffness of the central portion and the rim portion, respectively, is achieved by forming said portion as integrated sections having different thickness.

The reduced thickness of the rim portion, i.e. the lower stiffness, provides a flexibility to the rim portion that improves the sealing properties of the occluder.

The central portion 11 has a maximum thickness Tcp which gives the central portion an improved stiffness to carry a retracting load, while the rim portion 12 has a maximum thickness of Trp. Trp is typically approximately 1/2 of Tcp or less, and preferably about 1/3 of Tcp. A typical value of Tcp is about 1 mm.

The width Wcp of the central portion 11 should be less than the inner diameter of the introducing tool used to insert the occluder in the vessel, in order to allow the occluder to be folded and placed within the introducing tool. The overall width of the occluder (i.e. Wcp + 2*Wrp) is typically about 50% larger than the inner diameter of the introducing tool.

By inserting the occluder into the vessel in such a way that the respective end portions of the elongated central portion abut against the longitudinally situated puncture edge areas 8 of the inner vessel wall, a retracting force applied to the occluder will mainly be absorbed by those areas 8 having the highest strength against rupture. Preferably, the rim portion is tapered outwards towards its periphery in order improve its flexibility and to provide an essentially step free inner junction against the vessel wall.

When urging the intra-arterial occluder towards the inner wall of the vessel, the rim portion covers the areas surrounding the puncture, thereby sealing the puncture against blood penetration. Due to the elongated shape of the occluder, i.e. its extension along the length of the vessel, a considerably more safe sealing will be obtained compared to a conventional circular sealing.

In addition, the elongated shape of the present occluder provides a self-guiding effect in that the outer ends of the occluder will align themselves in the vessel as they are drawn towards the vessel wall.

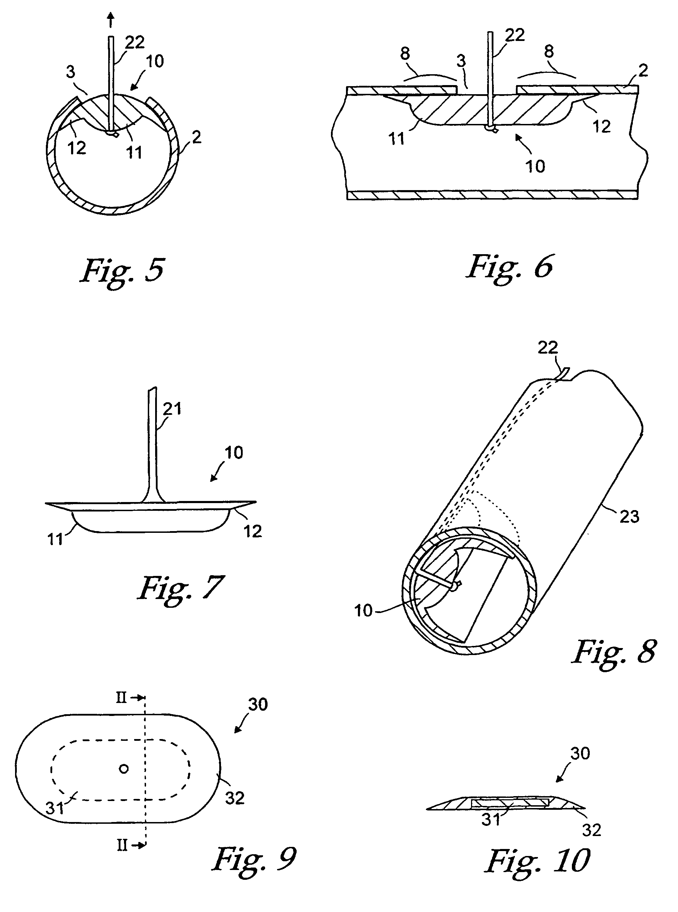

The position of an inserted occluder according to the invention is generally shown in the cross sectional views of Figs. 5 and 6, shown a crosswise (Fig. 5) and a lengthwise (Fig. 6) section. The occluder 10 abuts against the vessel wall 2 due to a retracting force in the retracting means 22. The rim portion 12 seals the puncture, and the ends sections of the central portion 11 rests against the comparatively strong areas 8, as described above.

Preferably, the central portion and the rim portion are integrated, allowing for easy one-step manufacturing.

Means for applying the retracting force are provided in the central portion of the occluder. For example, as is shown in Fig. 1, 2, 5 and 6, a through hole 13 is provided through which a string 22 could be passed and secured with a knot, a drop of an adhesive or a melted plastic, or similar. The occluder is urged towards the vessel wall by simply pulling the string. In Fig. 7 is shown another embodiment of a pulling device, namely a stem 21 protruding from the central portion 11 of the occluder 10. The stem is integrally formed with the occluder, and could optionally be provided with a saw-tooth profile (not shown) for mating with an optional extra-arterial occluder.

An occluder according to the invention is typically manufactured by injection molding, and is formed of any suitable flexible and biodegradable material.

The intra-arterial occluder and its retracting means is inserted into the vessel using an insertion tool similar to any suitable conventional tool, such as a tool described in any of the prior art documents referred to above. Thus, as is illustrated in Fig. 8, the occluder 10 according to the invention is folded along its longitudinal direction and is, together with its retracting means 22, inserted into a generally tubular tool 23. Then, the tool is inserted through the puncture and the occluder is pushed out completely into the vessel with its longitudinal direction essentially in the longitudinal direction of the vessel. The tool is removed and, using the retracting means, the occluder is urged against the inner vessel wall thereby sealing the puncture to stop blood from exiting through the puncture.

The pulling force of the retracting means is maintained by any conventional means such as an adhesive applied to the skin of the patient (as described in US 4,744,364) or an extra-arterial occluder (as described in US 5,350,399 and US 5,342,393).

Thus, with an occluder according to the present invention a tight sealing of a puncture in a vessel is obtainable. With the occluder of the invention, a lower sealing force is required compared to a case of a sealing means outside the vessel, since the blood pressure in the vessel acts on the occluder to urge it against the vessel wall.

The elongated structure of the occluder of the present embodiment, with its elongated thick central portion, ensures improved safety from ripping the edge of the puncture. At the same time, it is readily foldable for insertion into an introducer.

Although shown having a generally oval shape, the outer shape of the occluder could be any elongated shape ensuring a proper sealing of the puncture and, at the same time, allowing the thicker central portion to rest at the comparatively strong areas around the puncture, depicted with 8 in Fig. 4, or an essentially symmetrical shape as circular. However, the oval shape is preferred since it is assessed to limit the risk of injuring the vessel wall.

The difference in stiffness between the central portion and the rim portion of the occluder could also be achieved by other means than different thickness. For example, a second embodiment of an occluder 30 according to the present invention is shown in figs. 9 and 10, wherein the rim portion 32 is similar to the rim portion 12 of the first embodiment, but the higher stiffness of the central portion 33 is achieved with an insert 31 of a stiffer bioabsorbable material than the material of the rim portion 32. Of course, the central portion could be made entirely of the material having higher material stiffness (i.e. the stiffer material not being an insert but forming the central portion in itself), in which case the rim portion should be separate and attached to the central portion for example with an adhesive.

It is clear that the present invention may be varied in many ways with respect to the detailed description above. Such variations are not to be regarded as a departure from the scope of the invention, and all such modifications as would be clear to one skilled in the art are intended to be included within the scope of the following claims.

## Claims

1. An occluder (10; 30) for intra-arterial sealing a puncture in a vessel, the occluder being held against the vessel wall by applying a retraction force from outside the vessel to a central portion (11; 31) of the occluder, wherein said central portion (11; 31) is of an elongated shape having two longitudinal edges, said central portion having a length (Lcp) that exceeds the diameter of the puncture in the vessel direction and a width less than the diameter of the puncture in a direction transverse to the vessel direction and **characterized in that** the occluder (10;30) further comprises a rim portion (12; 32) provided at least along each longitudinal edge of the central portion; said central portion having higher structural stiffness than the rim portion, and **in that** the occluder is provided with a generally flat surface (14), extending over the central portion and the rim portion, for contact with the inner wall of the vessel around the puncture.

2. The occluder according to claim 1, wherein the higher stiffness of said central portion is achieved by providing the central portion (11) with a maximum thickness (Tcp) at least twice the maximum thickness (Trp) of said rim portion (12).

3. The occluder according to claim 2, wherein said rim portion (12) has a maximum thickness (Trp) not exceeding 1/3 of the maximum thickness (Tcp) of said central portion (11).

4. The occluder according to claim 1, wherein the higher stiffness of said central portion is achieved by providing the central portion (31) from a material having a higher material stiffness than the material of said rim portion (32).

5. The occluder according to anyone of the previous claims, wherein said central portion (11; 31) has a length (Lcp) approximately 50% longer than the diameter of the puncture in the vessel direction.

6. The occluder according to anyone of the previous claims, wherein said rim portion (12; 32) extends around the entire central portion (11; 31).

7. The occluder according to anyone of the previous claims, wherein said central portion (11; 31) is oval shaped.

8. The occluder according to anyone of the previous claims, wherein said rim portion (12; 32) is tapered outwards to its periphery.

9. The occluder according to anyone of the previous claims, wherein said central portion (11) includes means (13, 22; 21) for applying a sealing force.

## Patentansprüche

1. Verschluss (10; 30) zum Verschließen einer Punktur in einem Blutgefäß innerhalb einer Arterie, wobei der Verschluss gegen die Gefäßwand gehalten wird, indem eine Rückzugskraft von außerhalb des Gefäßes auf einen mittleren Bereich (11; 31) des Verschlusses aufgebracht wird, wobei der besagte mittlere Bereich (11; 31) eine längliche Gestalt mit zwei längsverlaufenden Kanten und eine Länge (Lcp) hat, die den Durchmesser der Punktur in Richtung des Gefäßes überschreitet, und eine Breite, die geringer ist als der Durchmesser der Punktur in einer Richtung quer zur Gefäßrichtung,
**dadurch gekennzeichnet,**
**dass** der Verschluss (10; 30) außerdem einen Randbereich (12; 32) aufweist, der zumindest entlang jeder längsverlaufenden Kante des mittleren Bereichs vorgesehen ist, wobei der mittlere Bereich eine größere strukturelle Steifigkeit hat als der Randbereich, und
**dass** der Verschluss mit einer im Allgemeinen flachen Oberfläche (14) versehen ist, die sich über den mittleren Bereich und den Randbereich hinüber erstreckt, für einen Kontakt mit der Innenwand des Gefäßes um die Punktur herum.

2. Verschluss nach Anspruch 1, bei welchem die höhere Steifigkeit des besagten mittleren Bereichs erzielt wird, indem der mittlere Bereich (11) mit einer maximalen Dicke (Tcp) versehen wird, die zumindest zweimal so dick ist wie die maximale Dicke (Trp) des Randbereichs (12).

3. Verschluss nach Anspruch 2, bei welchem der Randbereich (12) eine maximale Dicke (Trp) hat, die ein Drittel der maximalen Dicke (Tcp) des mittleren Bereichs (11) nicht überschreitet.

4. Verschluss nach Anspruch 1, bei welchem die höhere Steifigkeit des mittleren Bereichs erzielt wird, indem der mittlere Bereich (31) aus einem Material gemacht wird, dass eine höhere Materialsteifigkeit hat als das Material des Randbereichs (32).

5. Verschluss nach einem der vorangehenden Ansprüche, bei welchem der mittlere Bereich (11; 31) eine Länge (Lcp) hat, die ungefähr 50% länger ist als der Durchmesser der Punktur in der Gefäßrichtung.

6. Verschluss nach einem der vorangehenden Ansprüche, bei welchem sich jeder Randbereich (12; 32) um den gesamten mittleren Bereich (11; 31) herum erstreckt.

7. Verschluss nach einem der vorangehenden Ansprüche, bei welchem der mittlere Bereich (11; 31) oval gestaltet ist.

8. Verschluss nach einem der vorangehenden Ansprüche, bei welchem sich der Randbereich (12; 32) hin zu seinem Außenumfang nach außen verjüngt.

9. Verschluss nach einem der vorangehenden Ansprüche, bei welchem der mittlere Bereich (11) Mittel (13; 22; 21) zum Aufbringen einer Verschlusskraft beinhaltet.

## Revendications

1. Dispositif d'occlusion (10 ; 30) destiné à la fermeture intra-artérielle d'une perforation d'un vaisseau, le dispositif d'occlusion étant maintenu contre la paroi du vaisseau en appliquant une force de rétraction depuis l'extérieur du vaisseau vers une partie centrale (11 ; 31) du dispositif d'occlusion, dans lequel ladite partie centrale (11 ; 31) est d'une forme allongée présentant deux bords longitudinaux, ladite partie centrale ayant une longueur (Lcp) qui dépasse le diamètre de la perforation dans la direction du vaisseau et une largeur inférieure au diamètre de la perforation dans une direction transversale à la direction du vaisseau, et **caractérisé en ce que** le dispositif d'occlusion (10 ; 30) comprend en outre une partie de rebord (12 ; 32) disposée au moins le long de chaque bord longitudinal de la partie centrale ; ladite partie centrale ayant une rigidité structurale plus élevée que la partie de rebord, et **en ce que** le dispositif d'occlusion est doté d'une surface globalement plate (14), s'étendant sur la partie centrale et la partie de rebord, pour un contact avec la paroi intérieure du vaisseau autour de la perforation.

2. Dispositif d'occlusion selon la revendication 1, dans lequel la rigidité plus élevée de ladite partie centrale est obtenue en donnant à la partie centrale (11) une épaisseur maximale (Tcp) correspondant à au moins deux fois l'épaisseur maximale (Trp) de ladite partie de rebord (12).

3. Dispositif d'occlusion selon la revendication 2, dans lequel ladite partie de rebord (12) a une épaisseur maximale (Trp) ne dépassant pas 1/3 de l'épaisseur maximale (Tcp) de ladite partie centrale (11).

4. Dispositif d'occlusion selon la revendication 1, dans lequel la rigidité plus élevée de ladite partie centrale est obtenue en créant la partie centrale (31) à partir d'un matériau présentant une rigidité de matériau plus élevée que le matériau de ladite partie de rebord (32).

5. Dispositif d'occlusion selon l'une quelconque des revendications précédentes, dans lequel ladite partie centrale (11 ; 31) a une longueur (Lcp) approximativement 50 % plus longue que le diamètre de la perforation dans la direction du vaisseau.

6. Dispositif d'occlusion selon l'une quelconque des revendications précédentes, dans lequel ladite partie de rebord (12 ; 32) s'étend autour de toute la partie centrale (11 ; 31).

7. Dispositif d'occlusion selon l'une quelconque des revendications précédentes, dans lequel ladite partie centrale (11 ; 31) est de forme ovale.

8. Dispositif d'occlusion selon l'une quelconque des revendications précédentes, dans lequel ladite partie de rebord (12 ; 32) est effilée vers l'extérieur vers sa périphérie.

9. Dispositif d'occlusion selon l'une quelconque des revendications précédentes, dans lequel ladite partie centrale (11) comprend un moyen (13, 22 ; 21) destiné à appliquer une force d'étanchement.
